## Europäisches Patentamt

## European Patent Office

## Office européen des brevets

(11) Veröffentlichungsnummer: **0 308 637**
**A1**

## EUROPÄISCHE PATENTANMELDUNG

(21) Anmeldenummer: 88112688.2

(22) Anmeldetag: 04.08.88

(51) Int. Cl.4: **A61K 9/48 , A61J 3/07**

(30) Priorität: **21.08.87 DE 3727894**

(43) Veröffentlichungstag der Anmeldung:
**29.03.89 Patentblatt 89/13**

(84) Benannte Vertragsstaaten:
**AT BE CH DE ES FR GB GR IT LI LU NL SE**

(71) Anmelder: **Stephan, Dieter, Dr.**
**Gehrenwaldstrasse 35A**
**D-7000 Stuttgart(DE)**

Anmelder: **Stephan, Günter**
**Happoldstrasse 48**
**D-7000 Stuttgart(DE)**

(72) Erfinder: **Stephan, Dieter, Dr.**
**Gehrenwaldstrasse 35A**
**D-7000 Stuttgart(DE)**
Erfinder: **Stephan, Günter**
**Happoldstrasse 48**
**D-7000 Stuttgart(DE)**

(74) Vertreter: **Kastner, Hermann, Dipl.-Ing.**
**Osterholzallee 89**
**D-7140 Ludwigsburg(DE)**

(54) **Kapsel für pharmazeutisch wirksame Inhaltsstoffe einer Droge.**

(57) Bei der Kapsel (13) für pharmazeutisch wirksame Inhaltsstoffe einer Droge sind die Inhaltsstoffe (L) lipophiler Phase und die Inhaltsstoffe (P) polarer Phase in je einem eigenen Aufnahmeteil (25; 26) der Kapsel (13) untergebracht. Wenigstens eine Wand der Aufnahmeteile (25; 26) ist so beschaffen, daß eine Vermischung der Inhaltsstoffe (L; P) beider Phasen vermieden wird.

## Fig. 3

EP 0 308 637 A1

# Kapsel für pharmazeutisch wirksame Inhaltsstoffe einer Droge

Bei der Herstellung pharmazeutischer Produkte auf der Basis pflanzlicher oder tierischer Drogen werden die pharmazeutisch wirksamen Inhaltsstoffe aus der betreffenden Droge extrahiert. Diese Extraktion ist notwendig, um die Droge auf bestimmte Mindestgehalte an bestimmten Inhaltsstoffen standardisieren zu können. Die Extraktion ist häufig auch deswegen erforderlich, um unwirksame oder unverträgliche Anteile der Droge in ihrem Ausgangszustand, wie beispielsweise radioaktive Nukleide, Pflanzenschutzmittelrückstände, wie Pestizide, Herbizide usw., entfernen zu können. Daneben oder darüber hinaus kann eine Extraktion auch deswegen erforderlich sein, um die pharmazeutisch wirksamen Inhaltsstofe in einer Konzentration zu gewinnen, durch die die Einnahme der von den Standardmonographien vorgeschriebenen Drogenmenge überhaupt erst ermöglicht oder zumindest erleichtert wird.

Die Extraktionsbehandlung der Drogen erfolgt üblicherweise mit unterschiedlichen Lösungsmitteln. Die Wahl des Lösungsmittels hängt weitgehend von der Struktur der wirksamen Inhaltsstoffe ab. Bei Inhaltsstoffen polarer Phase wird die Extraktion mit ·Wasser und/oder mit Wasser-Äthylalkohol-Gemischen durchgeführt. Bei lipophilen Inhaltsstoffen erfolgt die Extraktion mit Pflanzenölen, mit Neutralölen oder mit Kohlendioxid im Hochdruckextraktionsverfahren (Destraktion).

Bei manchen Drogen sind pharmazeutisch wirksame Inhaltsstoffe sowohl polarer, wie auch lipophiler Phase vorhanden. Zur gemeinsamen Verabreichung dieser Inhaltsstoffe müssen sie in den durch die Standardmonographien vorgeschriebenen eng begrenzten Mengenverhältnissen miteinander gemischt werden. Das Mischen der Inhaltsstoffe, die entweder in wässriger Form oder in flüssiger Form in einem Wasser-Äthylalkohol-Gemisch vorliegen oder die nach dem Abdampfen der Lösungsmittel in getrockneter Form vorliegen, mit den Inhaltsstoffen lipophiler Phase in der Form einer Ölemulsion zum fertigen Arzneimittel ist allenfalls mit einem großen technischen Aufwand zu erreichen und manchmal sogar überhaupt nicht möglich. Um diese Schwierigkeiten zu überwinden ist es bekannt, die ölige Phase in Mikrokapseln einzuschließen und diese als eine Art festes Granulat mit den Trockenextrakten der polaren Phase zu mischen. Neben einem hohen technischen und finanziellen Aufwand treten analytische Probleme auf, im Fertigpräparat die einzelnen Bestandteile nachzuweisen und zu bestimmen.

Der in den Ansprüchen 1, 2 und 3 angegebenen Erfindung liegt die Aufgabe zugrunde, eine Darreichungsform zu schaffen, die mit geringerem technischem und/oder finanziellem Aufwand verwirklicht werden kann und bei der im Fertigpräparat die pharmazeutisch wirksamen Inhaltsstoffe sowohl der lipophilen Phase, wie auch der polaren Phase miteinander vereinigt sind, ohne daß sie sich gegenseitig in unerwünschter Weise beeinflussen können.

Dadurch, daß nach Anspruch 1 eine Kapsel mit zwei Aufnahmeteilen verwendet wird, bei der die Wand wenigstens eines der beiden Aufnahmeteile die Inhaltsstoffe beider Phasen in keiner Richtung durchtreten läßt, oder dadurch, daß bei einer Kapsel nach Anspruch 2 die Wand jedes der beiden Aufnahmeteile den zugeordneten Inhaltsstoff nicht austreten läßt, oder dadurch, daß bei einer Kapsel nach Anspruch 3 die Trennwand für die Inhaltsstoffe beider Phasen undurchlässig ist, können diese Inhaltsstoffe sehr leicht gemeinsam verabreicht werden. Die getrennte Behandlung der Inhaltsstoffe beider Phasen beim Einbringen in eine dieser Kapseln erfordert keinen sehr hohen technischen und/oder finanziellen Aufwand. Dadurch lassen sich auch von solchen Drogen die Inhaltsstoffe unterschiedlicher Phase mit erträglichem Aufwand verarbeiten und verabreichen, die ihrer Natur nach nicht unmittelbar miteinander gemischt werden können.

Bei einer Ausgestaltung der Erfindung nach Anspruch 4 können die Inhaltsstoffe der einen Phase zunächst in einem eigenen Behältnis, beispielsweise in form einer Weichgelatinekapsel, untergebracht werden und dieses Behältnis dann in die Kapsel, beispielsweise in Form einer Hartgelatinekapsel, eingebracht werden, in die entweder vorher oder nachher die Inhaltsstoffe der anderen Phase eingebracht wurden bzw. werden. Bei einer Ausgestaltung der Erfindung nach Anspruch 5 werden die Inhaltsstoffe beider Phasen je für sich in eine Abteilung einer Doppelkammerkapsel eingebracht, die durch den Steg, der beide Abteilungen miteinander verbindet, als Fertigpräparat trotzdem eine Einheit bildet. Bei einer Ausgestaltung der Erfindung nach Anspruch 6 können die Inhaltsstoffe beider Phasen auch bei voneinander unterschiedlicher Konsistenz zunächst je in ein eigenes Behältnis eingebracht werden und die beiden Behältnisse anschließend in die sie gemeinsam aufnehmende Kapsel, insbesondere in Form einer Hartgelatinekapsel, eingebracht werden, die das Fertigpräparat bildet.

Im folgenden wird die Erfindung anhand mehrerer in der Zeichnung dargestellter Ausführungsbeispiele näher erläutert. Es zeigen:

Fig. 1 eine Ansicht eines ersten Ausführungsbeispieles der Kapsel;

Fig. 2 einen Längsschnitt eines zweiten Ausführungsbeispieles der Kapsel;

Fig. 3 einen Längsschnitt eines dritten Ausführungsbeispieles der Kapsel;

Fig. 4 einen Längsschnitt eines vierten Ausführungsbeispieles der Kapsel.

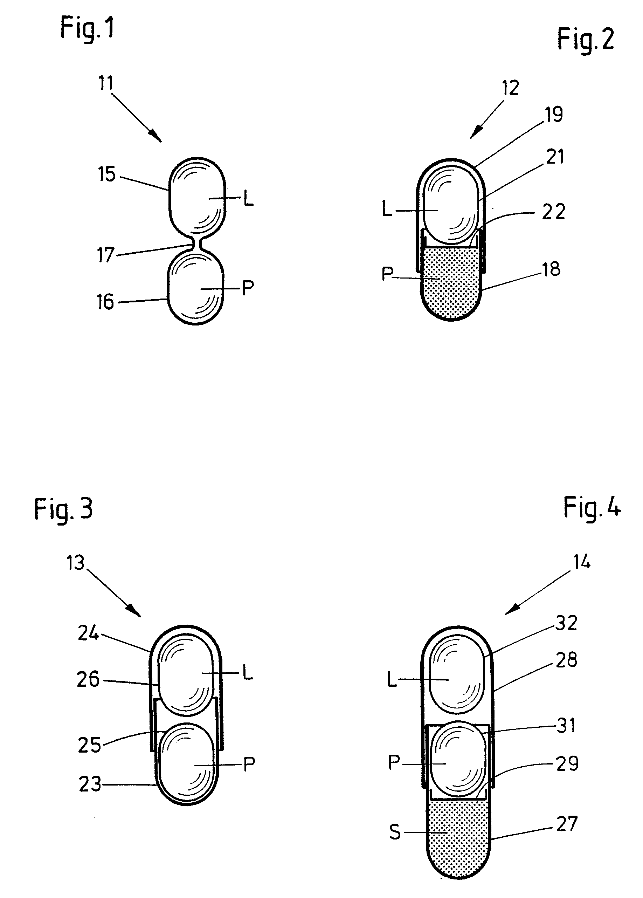

Die aus Fig. 1 ... 4 ersichtlichen Kapseln 11 ... 14 sind für die Aufnahme von pharmazeutisch wirksamen Inhaltsstoffen lipophiler Phase und polarer Phase einer Droge bestimmt. Zu ihrer Unterscheidung sind die lipophilen Inhaltsstoffe mit "L" und die polaren Inhaltsstoffe mit "P" gekennzeichnet. Bei der Kapsel 14 ist noch eine dritte Art sonstiger Inhaltsstoffe vorhanden, die mit "S" bezeichnet sind.

Die aus Fig. 1 ersichtliche Kapsel 11 ist als Weichgelatinekapsel ausgebildet. Sie weist je ein in sich abgeschlossenes Behältnis 15 und 16 auf, die bei der Herstellung der Kapsel 11 aus dem Werkstoff für Kapseln geformt werden, wobei die Wandungen der beiden Behältnisse 15 und 16 noch während des Füllvorganges in sich dicht verschlossen werden. Bei diesem Herstellungsvorgang wird zwischen den beiden kugelförmigen oder langrunden Behältnissen 15 und 16 aus demselben Werkstoff wie diese zugleich ein Steg 17 angeformt oder, genauer ausgedrückt, zwischen den beiden Behältnissen 15 und 16 stehengelassen. Dieser Steg 17 verbindet die beiden Behältnisse 15 und 16 zu einer Doppelkammerkapsel.

Die Kapsel 11 wird vor allem dann verwendet, wenn die Inhaltsstoffe P und L in fließfähiger Form, d.h. in flüssiger oder in pastöser Form, vorliegen.

Die aus Fig. 2 ersichtliche Kapsel 12 ist dreiteilig ausgebildet. Der äußere Teil ist eine Hartgelatinekapsel mit den beiden Kapselteilen 18 und 19. Der innere Teil ist als in sich abgeschlossenes Behältnis 21 in Form einer Weichgelatinekapsel ausgebildet.

Das Behältnis 21 nimmt vorzugsweise die lipophilen Inhaltsstoffe L auf, die im allgemeinen in fließfähiger Form, d.h. in flüssiger oder pastöser Form vorliegen. Von den beiden Kapselteilen 18 und 19 nimmt der beim Zusammenfügen innen gelegene Kapselteil 18 vorzugsweise die polaren Inhaltsstoffe P auf, und zwar insbesondere dann, wenn diese in rieselfähiger Form, also im allgemeinen in getrockneter Form, vorliegen. Die Handhabung des Kapselteils 18 nach dem Befüllen mit den polaren Inhaltsstoffen P wird dadurch erleichtert, daß ein Deckel 22 eingesetzt wird, der die Füllung im Kapselteil 18 festhält. Soweit die Füllung mit den polaren Inhaltsstoffen P trocken ist, genügt es, den Deckel 22 mit einer entsprechenden Passung herzustellen und einfach hineinzudrücken. Falls das nicht ausreichend sein sollte, insbesondere dann, wenn die Inhaltsstoffe P ebenfalls in fließfähiger Form vorliegen oder wenn sie aus anderen Gründen schon vor dem Verschließen der Kapsel 12, d.h. vor dem Aufstecken des zweiten Kapselteils 19, vor den Einflüssen der Umgebung geschützt werden sollen, dann kann der Deckel 22 auch eingeklebt oder eingeschweißt werden. In diesem Falle bildet der Deckel 22 eine feste Trennwand zwischen den beiden Aufnahmeteilen für die Inhaltsstoffe P und L. Wenn der polare Inhaltsstoff in thixotroper Form vorliegt oder wenn er durch kurzzeitiges Erwärmen verflüssigt werden kann und er dadurch in fließfähiger Form abgekühlt werden kann, wonach er wieder erstarrt, dann kann auf den Deckel 22 verzichtet werden.

Wenn nach dem Einfüllen der polaren Inhaltsstoffe P und gegebenenfalls nach dem Einsetzen des Deckels 22 auch noch das Behältnis 21 mit den lipophilen Inhaltsstoffen L eingebracht wurde, wird der äußere zweite Kapselteil 19 auf den ersten Kapselteil 18 aufgesteckt und die Kapsel damit verschlossen.

Die aus Fig. 3 ersichtliche Kapsel 13 ist vierteilig ausgebildet. Der äußere Teil ist wiederum eine Hartgelatinekapsel mit den beiden Kapselteilen 23 und 24. Im Inneren befinden sich die beiden in sich abgeschlossenen Behältnisse 25 und 26. Darin sind die lipophilen Inhaltsstoffe L und die polaren Inhaltsstoffe P je getrennt voneinander untergebracht. Für die beiden Behältnisse 25 und 26 bildet die Kapsel 13 eine gemeinsame Hülle.

Diese Ausführungsform der Kapsel kommt vor allem dann in Betracht, wenn die Inhaltsstoffe beider Phasen in fließfähiger Form vorliegen, die zwei in sich abgeschlossene Behältnisse erfordern, und dafür aber aus irgendwelchen Gründen die Ausführungsform der Kapsel 11 nach Fig. 1 nicht in Betracht kommt. Das kann z.B. dann der Fall sein, wenn die Inhaltsstoffe beider Phasen an unterschiedlichen Orten oder zu unterschiedlichen Zeiten verarbeitet werden müssen und unabhängig voneinander in ein Behältnis eingefüllt werden müssen.

Die aus Fig. 4 ersichtliche Kapsel 14 stellt eine Abwandlung der Kapsel 13 dar. Ihr äußerer Teil ist ebenfalls eine Hartgelatinekapsel mit den Kapselteilen 27 und 28. Im inneren Kapselteil 27 ist eine dritte Art Inhaltsstoffe S untergebracht. Wie schon zuvor bei der Kapsel 12, werden diese Inhaltsstoffe S - bei Bedarf - durch einen Deckel 29 festgehalten und gegenüber dem übrigen Innenraum der Kapsel 14 abgeschlossen. In diesem übrigen Innenraum befinden sich zwei in sich abgeschlossene Behältnisse 31 und 32 in Form je einer Weichgelatinekapsel. Darin sind, wie bei der Kapsel 13, die lipophilen Inhaltsstoffe L und die polaren Inhaltsstoffe P je getrennt voneinander untergebracht. Auch hier dient die Kapsel 14 als Hülle für die beiden Behältnisse 31 und 32 und zugleich als Aufnahmeteil für die Inhaltsstoffe S.

Diese Ausführungsform kommt vor allem für die gleichzeitige Verabreichung von drei verschiedenen Arten Inhaltsstoffe in Betracht. Dabei sind auch andere Abwandlungen möglich. Beispielsweise kann auch der äußere Kapselteil 28 unmittelbar mit Inhaltsstoffen in rieselfähiger oder thixotroper Form befüllt werden und erforderlichenfalls mit einem Deckel verschlossen werden. Es könnten aber auch drei (oder mehr) in sich abgeschlossene Behältnisse in Form von Weichgelatinekapseln in die Kapsel 14 als gemeinsame Hülle eingebracht werden.

**Ansprüche**

1. Kapsel für pharmazeutisch wirksame Inhaltsstoffe einer Droge,
**gekennzeichnet** durch die Merkmale:
- die Inhaltsstoffe (L) lipophiler Phase und die Inhaltsstoffe (P) polarer Phase sind in je einem Aufnahmeteil (21, 18; 19) der Kapsel (12) getrennt voneinander untergebracht,
- die Wand zumindest des einen Aufnahmeteils (21) ist für den Durchtritt der Inhaltsstoffe (L) beider Phasen undurchlässig.

2. Kapsel für pharmazeutisch wirksame Inhaltsstoffe einer Droge,
**gekennzeichnet** durch die Merkmale:
- die Inhaltsstoffe (L) lipophiler Phase und die Inhaltsstoffe (P) polarer Phase sind in je einem eigenen Aufnahmeteil (26; 25) der Kapsel (13) getrennt voneinander untergebracht,
- die Wand jedes der beiden Aufnahmeteile (25; 26) der Kapsel (13) ist in Bezug auf das Austreten der darin befindlichen Inhaltsstoffe (P; L) undurchlässig.

3. Kapsel für pharmazeutisch wirksame Inhaltsstoffe einer Droge,
**gekennzeichnet** durch die Merkmale:
- die Inhaltsstoffe (L) lipophiler Phase und die Inhaltsstoffe (P) polarer Phase sind in je einem eigenen Aufnahmeteil (15; 16) der Kapsel (11) getrennt voneinander untergebracht,
- die beiden Aufnahmeteile sind als Teilräume (15; 16) der Kapsel (11) ausgebildet, die mittels einer Trennwand (17) abgetrennt sind, die für den Durchtritt der Inhaltsstoffe (L; P) beider Phasen undurchlässig ist.

4. Kapsel nach Anspruch 1,
**gekennzeichnet** durch die Merkmale,
- der eine Aufnahmeteil ist als abgeschlossenes Behältnis (21) ausgebildet,
- der andere Aufnahmeteil wird durch die Kapsel (12) gebildet, die neben den zugeordneten Inhaltsstoffen (P) zugleich auch das eine Behältnis (21) aufnimmt.

5. Kapsel nach Anspruch 1 oder 2,
**gekennzeichnet** durch das Merkmal,
- die beiden Aufnahmeteile der Kapsel (11) werden durch je ein abgeschlossenes Behältnis (15; 16) gebildet, die beide mittels eines äußeren Steges (17) miteinander verbunden sind, der vorzugsweise aus dem gleichen Werkstoff wie die Behältnisse (15; 16) ist.

6. Kapsel nach Anspruch 2,
**gekennzeichnet** durch die Merkmale,
- die beiden Aufnahmeteil der Kapsel (13) werden durch je ein abgeschlossenes Behältnis (25; 26) für die zugeordneten Inhaltsstoffe (P; L) gebildet,
- die beiden Behältnisse (25; 26) sind von der Kapsel (13) als gemeinsame Hülle umschlossen.

# Fig.1

11

15

L

17

16

P

# Fig.2

12

19

21

L

22

P

18

# Fig. 3

13

24

26

L

25

23

P

# Fig. 4

14

32

28

L

31

29

P

S

27

Dr. D. Stephan, G. Stephan

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DERWENT PUBLICATIONS LTD, Datenbank: WPI; Ref.Nr. 78-24571a, London, GB; & NL-A-76 10 038 (TAPANAHONY) 09/09/1976 * Zusammenfassung * --- | 1,2 | A 61 K 9/48 A 61 J 3/07 |
| X | EP-A-0 211 079 (FUJISAWA) * Ansprüche 1-4; Seite 2, Zeilen 15-18; Seite 3, Zeilen 5-15 * ----- | 1,2 | |

**RECHERCHIERTE SACHGEBIETE (Int. Cl.4)**

A 61 K
A 61 J

Der vorliegende Recherchenbericht wurde für alle Patentansprüche erstellt

| Recherchenort | Abschlußdatum der Recherche | Prüfer |
|---|---|---|
| DEN HAAG | 06-12-1988 | SCARPONI U. |